Europäisches Patentamt

European Patent Office

Office européen des brevets

(19)

(11) Publication number: **0 257 458**
**A2**

# EUROPEAN PATENT APPLICATION

(21) Application number: 87111699.2

(22) Date of filing: 12.08.87

(51) Int. Cl.4: **A61K 7/50 , C01F 11/18**

(30) Priority: 22.08.86 JP 195161/86
23.02.87 JP 38017/87

(43) Date of publication of application:
**02.03.88 Bulletin 88/09**

(84) Designated Contracting States:
**DE FR GB**

(71) Applicant: **Matsuzaki, Akira**
**2-34-712 Kitakasai 4-chome Edogawa-ku**
**Tokyo(JP)**

Applicant: **Ozaki, Yukio**
**7-1-1407 Kitasuna 7-chome Kouto-ku**
**Tokyo(JP)**

(72) Inventor: **Matsuzaki, Akira**
**2-34-712 Kitakasai 4-chome Edogawa-ku**
**Tokyo(JP)**

(74) Representative: **Münzhuber, Robert,**
**Dipl.-Phys.**
**Patentanwalt Rumfordstrasse 10**
**D-8000 München 5(DE)**

(54) **Scrubbing agent and process for producing the same.**

(57) This invention provides a scrubbing agent comprising granulated calcite extracted from glacial limestone. A process for producing a scrubbing agent containing calcite as the main component, comprises the crushing step of crushing glacial limestone and the fractionating step of subjecting the thus-crushed glacial limestone to air fractionation to extract a fractionated portion containing calcite as the main component. In addition, another process for producing a scrubbing agent containing calcite as the main component, comprising: the crushing step of crushing glacial limestone; the suspending step of suspending the thus-crushed glacial limestone in water; the fractionating step of placing the thus-obtained suspension in a stationary manner to collect supernatant liquid; and the drying step of drying the supernatant liquid.

F I G. 1

EP 0 257 458 A2

Xerox Copy Centre

## SCRUBBING AGENT AND PROCESS FOR PRODUCING THE SAME

### BACKGROUND OF THE INVENTION AND PRIOR ART STATEMENT

The present invention relates to a scrubbing agent and a process for producing the same, and more particularly to an improvement in the scrubbing agent.

A variety of cosmetics have heretofore been used all over the world, and a so-called scrub type of cleansing preparation has recently attracted considerable attention.

The scrub type of cleansing preparation differs from a normal type of cleansing preparation in that a granulated scrubbing agent is added to the former.

In general, as compared with the cleansing of a body, that of a face further requires proper removal of sebaceous substances, a massage effect and effective removal of old horny substances from the surface of skin.

For this reason, a scrub type of cleansing preparation having a particle size which enables a "grinding" effect with respect to skin without giving harm thereto is used for the purpose of removing horny substances which are not easily removed merely with soap and water. Use of such a scrub type of cleansing preparation realizes clear and glossy skin and at the same time provides a stimulus to skin, thereby activating body metabolism. It is therefore possible to remove dirt from pores in the skin.

A scrubbing agent for use as such a scrub type of cleansing preparation has heretofore been selected from the group consisting of synthetic resins such as polyethylene powders and granulated particles, vegetable materials such as apricot seeds, coconut particles and almond flesh as well as minerals such as marble and crystalline particles.

Such a scrubbing agent is commonly mixed with a skin cleansing base having no strong stimulus to skin; for example, an amino acid surfactant such as N-acyl-L-glutamic acid salt and N-acyl-N-alkyl-8-alanine sodium salt as well as an imidazolinium betaine type ampholiytic surfactant or the like. This mixture enables the scrubbing agent to enclose and remove dirt, old horny substances and the like without involving the excessive stimulus of the scrubbing agent with respect to skin.

In use, the scrubbing cleansing preparation having the aforesaid composition is applied to skin in a proper amount and the skin is cleansed while massage is being performed on the skin by hand or fingers, thereby enabling achievement of the aforementioned various effects.

However, such prior-art scrub type of cleansing preparation involves disadvantage in that no desired effects may be completely obtained.

Although a variety of cleansing preparations containing different scrubbing agents are currently produced and marketed, use of such cleansing preparations does not enable satisfactory removal of dirt, skin components or the like in skin pores.

In addition, since the prior-art scrub type of cleansing preparation has a coarse particle size and contains edgy particles, if a user forcedly cleanses his/her face using such a cleansing preparation, the skin of the user may be hurt to an undesirable extent. Also, if a user having an allergic constitution employs the cleansing preparation, his/her skin may be roughened. Accordingly, since it is difficult to use the prior-art scrub type of cleansing preparation, a demand has arisen for the development of a superior scrub type of cleansing preparation.

It is therefore an object of the present invention to provide an improved scrub type of cleansing preparation and a process for producing the same, the inventive cleansing preparation capable of achieving superior massage and cleansing effects without adversely affecting the skin of the user.

### SUMMARY OF THE INVENTION

A scrubbing agent of the present invention comprises granulated calcite extracted from glacial limestone, the granulated calcite having a substantial particle size of 10 μm or less.

Also, in the scrubbing agent comprising the granulated calcite extracted from glacial limestone or in the scrubbing agent comprising the granulated calcite extracted from glacial limestone and having a substantial particle size of 10 μm or less, such granulated calcite has the following particle size composition:

| 32 - 42 Me | ; 0.0 - 15.0% |
| 42 - 60 Me | ; 0.0 - 15.0% |
| 60 - 80 Me | ; 0.0 - 20.0% |

2

0 257 458

80 - 100 Me ; 10.0 - 60.0%
100 - 150 Me ; 15.0 - 35.0%
150 - 200 Me ; 5.0 - 40.0%
200 Me or more ; 5.0 - 30.0%

The aforesaid scrubbing agent has a pH which is adjusted to a neutral or weakly acid level with the use of organic acid or fatty acid.

In another aspect of the present invention, a process for producing the aforesaid scrubbing agent comprises: the crushing step of crushing glacial limestone; and the fractionating step of subjecting the thus-crushed limestone to air fractionation to extract the thus-fractionated portion containing calcite as the main component.

In another aspect of the present invention, a process for producing the aforesaid scrubbing agent comprises: the crushing step of crushing glacial limestone; the suspending step of suspending the thus-crushed limestone in water; a the fractionating step of placing the suspension in a stationary manner to collect the resultant supernatant liquid; and the drying step of drying the thus-collected supernatant liquid.

The aforesaid process is characterized in that stirring is performed for thirty to forty minutes in the suspending step, the suspension being placed in a stationary manner for twenty to forty minutes in the fractionating step, and in addition, the suspending and separating steps being repeated a plurality of times.

As is evident from the foregoing, the scrub type of cleansing preparation in accordance with the present uses glacial limestone as its main row material.

As is well known, in an area where glaciers exist, rocks or similar are generally formed into edgeless and smooth minerals by virtue of the grinding effect of the glaciers for many years. Thus, glacial sedimentary rocks typically have a fine particle size and a roundish form. In this case, calcite is particularly low in hardness, and this constitutes one conspicuous feature of the present invention.

Accordingly, a scrubbing agent of the kind that is comprised of calcite extracted from glacial limestone is substantially prevented from providing an excessive stimulus to skin, and thus it is well suited for application to the skin of users having an allergic constitution.

In addition, the fineness of the particles of the aforesaid scrubbing agent facilitates the removal of wastes from pores in the skin.

Also, the calcite produces carbon oxide, depending on the pH or temperature of an environment in which it is present. This provides a proper stimulus to the skin and improves its massage effect to a remarkable extent.

More specifically, skin is typically weakly acid, and in addition carbon dioxide is generated from calcite by friction heat produced by massage which is performed during the use of the scrubbing agent. This produces a synergistic effect together with the massage effect provided by the action of hands or fingers.

The aforesaid scrubbing agent is dissolved in water or mixed with beef tallow added thereto to prepare a scrub type of cleansing preparation. When the thus-formed scrub type of cleansing preparation is applied to a face in a proper amount and massage is then performed on the face, such cleansing preparation has been found to exhibit a remarkably good ability with respect to face cleansing as compared with the prior-art cleansing preparations. In addition, it has been confirmed that body metabolism is activated.

In accordance with the prior art, an improvement in the ability with respect to face cleansing may result in an excessive stimulus to skin and the occurrence of skin irritations since the prior-art cleansing preparation excessively removes not only lipide derived from glandulae cutes but also that derived from a horny layer which should not be excessively removed. However, the present cleansing preparation substantially prevents the occurrence of such problems, and this indicates that the present cleansing preparation has superior characteristics as a scrub type of cleansing preparation.

In a process for producing a scrubbing agent in accordance with the invention, glacial limestone is crushed in the crushing step.

In consequence, crushed substances containing desired calcite and other impurities are obtained.

In the next fractionating step, the crushed substances are subjected to air fractionation.

Since the specific gravity of calcite is smaller than that of many other impurities and its particle size is remarkably fine, the particles of calcite are blown away and are thus separated from the remaining components.

It is therefore possible to obtain a scrubbing agent containing calcite as the main component.

In another process for producing a scrubbing agent in accordance with the invention, glacial limestone is crushed in the crushing step, and thus crushed substances containing calcite and impurities are obtained.

In the next suspending step, the calcite and the impurities are suspended or dissolved in water.

3

In the fractionating step, the obtained suspension is placed in a stationary manner. Since calcite is difficult to dissolve in water but has a fine particle size and small specific gravity, the calcite component remains in supernatant liquid.

In the subsequent drying step, the aforesaid supernatant liquid is dried, thereby obtaining a scrubbing agent containing calcite as the main component.

As described above, in accordance with the present invention, calcite including fine and roundish particles which are originally contained in glacial limestone can be successfully extracted without changes in shape nor quality of the same.

## BRIEF DESCRIPTION OF THE DRAWINGS

Fig. 1 is a chart showing the result of powder X-ray diffraction of a scrubbing agent used in a cleansing preparation in accordance with the present invention;

Fig. 2A is a photograph showing at a $1,000^{\times}$ magnification the particle structure of the scrubbing agent used in the cleansing preparation in accordance with the present invention;

Fig. 2B is a photograph showing at a $4,000^{\times}$ magnification the particle structure of the scrubbing agent used in the cleansing preparation in accordance with the present invention;

Fig. 3A is a photograph showing at a $1,000^{\times}$ magnification the particle structure of calcite ($CaCO_3$) which has heretofore been obtained;

Fig. 3B is a photograph showing at a $4,000^{\times}$ magnification the particle structure of calcite ($CaCO_3$) which has heretofore been obtained;

Fig. 4 is a schematic illustration used as an aid in explaining a process for extracting a scrubbing agent from limestone in accordance with the present invention;

Fig. 5 is a schematic illustration used as an aid in explaining another process for extracting a scrubbing agent from limestone in accordance with the present invention.

## DETAILED DESCRIPTION OF PREFERRED EMBODIMENTS

Preferred embodiments of a scrub type of cleansing preparation of the invention will be described in detail below.

## SCRUBBING AGENT

The following is a description of a scrubbing agent which constitutes one feature of the present invention.

The scrubbing agent used in the invention is originally derived from glacial limestone which was found in the Iranian region.

The present inventors sought a material suitable for the desired scrubbing agent among natural substances from the viewpoint of safety required for cleansing preparations, and conducted tests on various minerals and considered the suitability of them for the scrubbing agent.

In consequence, the present inventors found that calcite ($CaCO_3$) extracted from the limestone produced in the Iranian region is suitable for use as a scrubbing agent owing to its superior characteristics.

The result of analysis of limestone produced in the Iranian region is listed below.

CaO ; 54.45%
$Al_2O_3$ ; 0.43%
$SiO_2$ ; 1.35%
MgO ; 0.40%
$Fe_2O_3$ ; 0.18%
MnO ; 0.01%
SrO ; 0.02%
$Na_2O$ ; 0.04%
$CO_2$ ; 42.68%
F ; 0.014%
Ba ; not detected
Pb ; 1 ppm or less

As ; 5 ppm or less

The methods of testing are as follows.

CaO ; EDTA Titration Method

$Al_2O_3$, MgO, $Fe_2O_3$, MnO, SrO ; Atomic Absorption

Photometry

$SiO_2$ ;gravimetic Method

$Na_2O$ ;Flame Photometry

$CO_2$ ;Neutralizatin Titration Method

Specific Gravity;Pycnometer

Ba .;Flame Reaction Method

Pb ;Dighizone Extraction Absorption

Photometry

As ;Diethyldithiocarbemic Acid Silver

Absorption Photometry

F :Lanthanum Alizarine Compton

Absorption Photomety

The respective tests upon F, dry weight, Ba, Pb, As and specific gravity were conducted in accordance with a process for analyzing precipitated calcium carbonate under the cosmetic raw material standards prevailing in Japan.

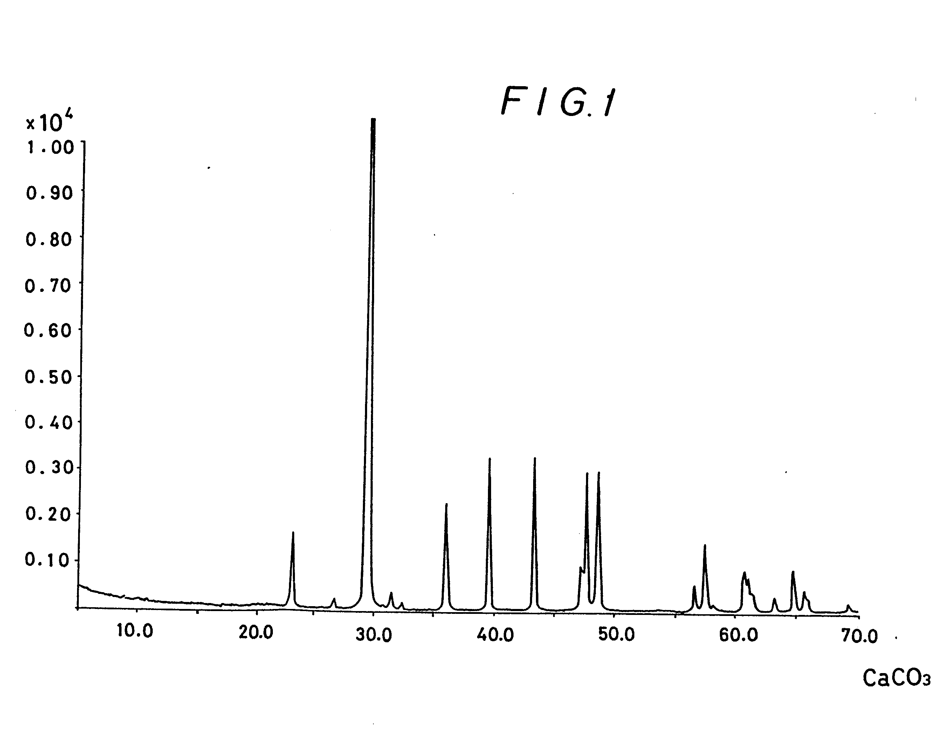

In addition, the prevent inventors conducted powder X-ray diffraction, and the result is shown in Fig. 1.

As can be seen from Fig. 1, the crystalline phase constituting the scrubbing agent is the single phase of $CaCO_3$.

The analysis was made by means of an fully automatic powder X-ray diffraction device PW-1700 (manufactured by Phillips) under the conditions of a Cu bulb, a voltage of 40kV, a current of 60mA, a scanning speed of 2°/min and a scanning range of 5° - 70° = 2.

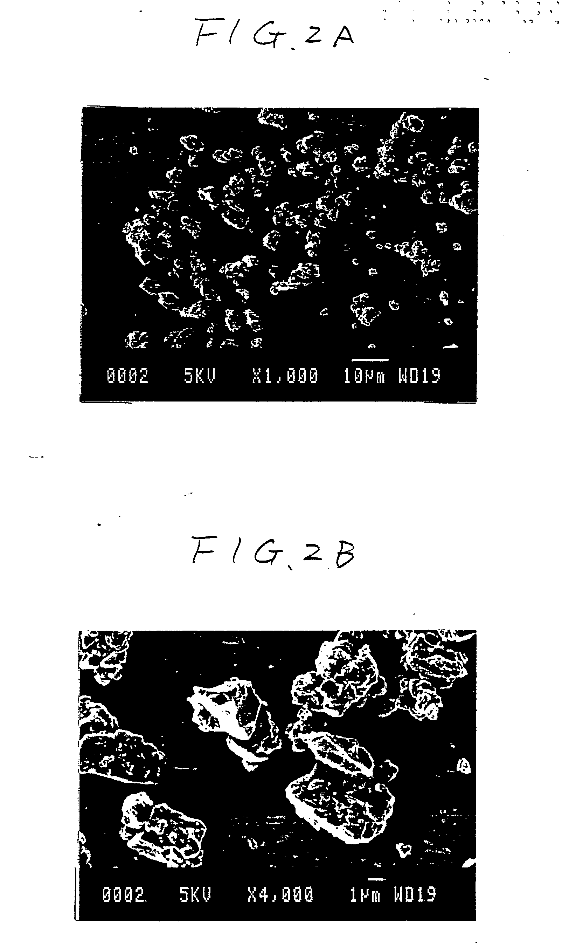

Fig. 2A is a photograph showing at a 1,000$^\times$ magnification the particle structure of a scrubbing agent produced in Iran with Fig. 2B another photograph showing the same at a 4,000$^\times$ magnification.

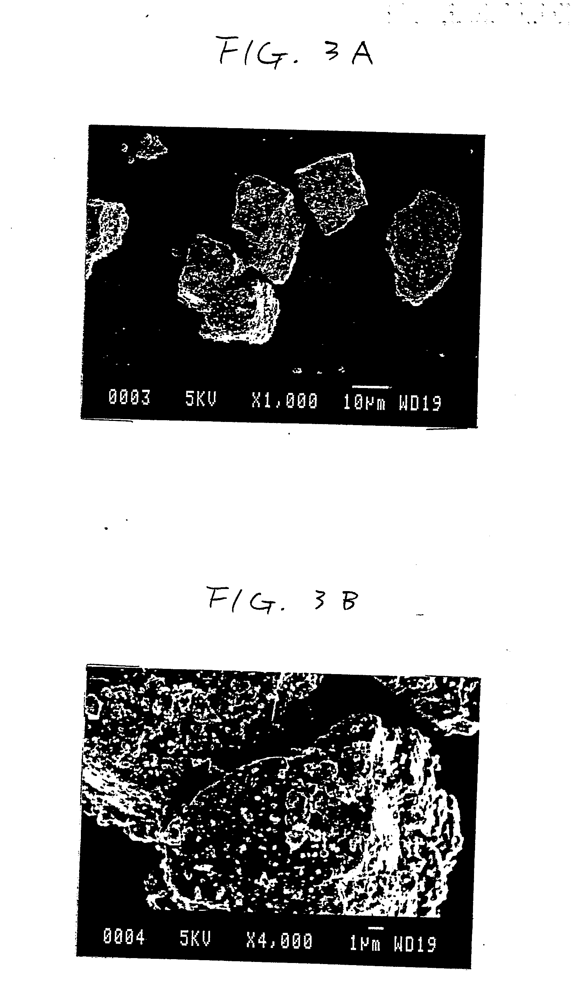

In contrast, Fig. 3A is a photograph showing at a 1,000$^\times$ magnification the particle structure of calcite ($CaCO_3$) which has heretofore been used with Fig. 3B another photograph showing the same at a 4,000$^\times$ magnification.

With reference to Figs. 2A to 3B, the scrubbing agent produced in Iran is compared with $CaCO_3$ which has heretofore been used. The scrubbing agent produced in Iran contains particles having a roundish and irregular form, and the particle diameter is 10 μm or less. Referring to Fig. 2B, traces of exfoliation are found on the particle surfaces, and this indicates that the hardness of the particles is low. For this reason, it is considered that the particles have a roundish and irregular form with recesses.

In contrast, $CaCO_3$ which is conventionally used consists of large flocks each having a diameter of 20 to 50 μm, and many of them assume a prismatic shape in contrast to the scrubbing agent produced in Iran.

The above-described difference between their physical properties has a great influence upon a specific surface area which determines the effects of the particles, such as a massage effect, a cleansing effect or the like.

More specifically, if massage is performed with the use of the scrubbing agent produced in Iran, strings of refuse are produced unlike other scrubbing agents, but no scrubbing agent remains in pores in skin. This is because, since the scrubbing agents produced in Iran typically have irregular form, it is considered that wastes, sebaceous substances or the like of skin get caught in the recesses of the agents, and are thus formed into agglomerates therein. $CaCO_3$ which is conventionally used is not accompanied by such a phenomenon.

The scrubbing agent produced in Iran having the aforesaid features may be regarded as one of hexagonal system calcite. As is known, after an originally aqueous rock has been dissolved and secondarily precipitated in groundwater, the resultant precipitate is changed into a crystalline substance by the effect of the adjoining eruptive rock after the elapse of a long period of time and the crystalline substance is grown into the aforesaid scrubbing agent by virtue of the "grinding effect" of a glacier. In addition, it is considered that, since the particles are placed under high pressure for a long period of time, certain orientation is present in the crystalline structure of each particle, and this may result in the occurrence of the aforesaid exfoliation.

5

Also, about 40% of the present scrubbing agent is occupied by $CO_2$, and, during the use thereof, evaporation is caused by frictional heat because of its weak acidity. This provides a stimulus to associated capilaries and enhances its massage effect. Although this phenomenon is also extremely unique, it is considered that a proper amount of carbon dioxide may be generated since the surface areas of the particles contained in the present scrubbing agent are remarkably great because of their irregular shapes.

Accordingly, since the present scrubbing agent provides no excessive stimulus to the skin of the user, it can be applied to the skin of any of normal, dry and wet types.

As is event from the above noted result of analysis, any of the components of the present scrubbing agent meets the cosmetic raw material standards prevailing in Japan, and contains no noxious components.

Also, the aforesaid scrubbing agent contains alkaline $CaCO_3$ as the main component, and its pH is 9.5.

A typical cleansing preparation preferably has a neutral or weakly acid pH which is approximately equal to the pH of skin. It is therefore preferable to perform adjustment of the pH, as required, with the use of fatty acid such as oleic acid, stearic acid, hyaluronic acid and palmitic acid or organic acid such as citric acid, tartaric acid, succinic acid, edit acid and lactic acid. In this case, there is no risk that such pH adjustment affects the retentability of the present scrubbing agent with respect to carbon dioxide.

The following is a description of a process for extracting calcite from glacial limestone produced in Iran.

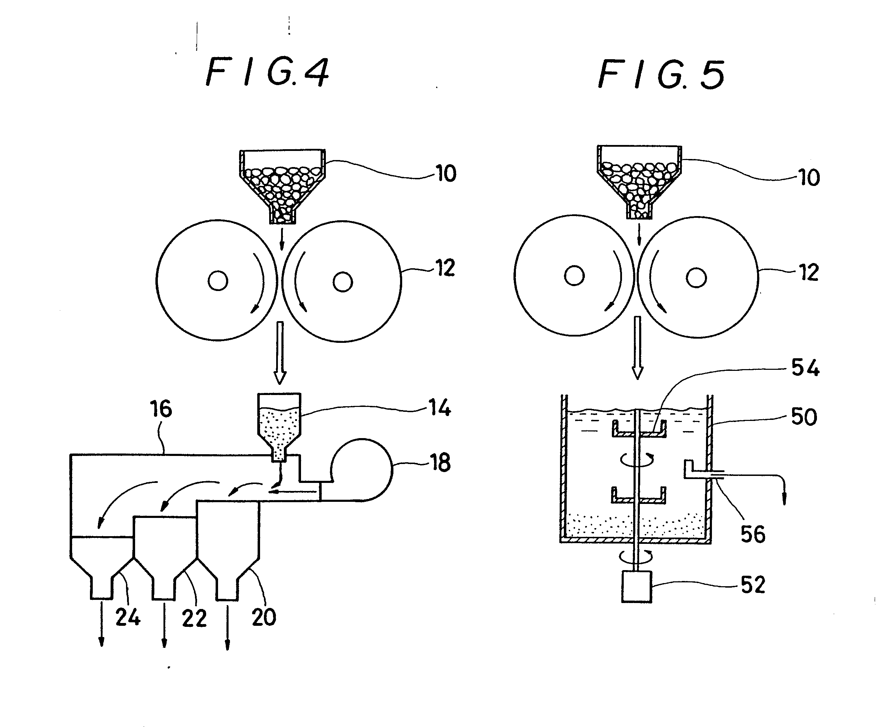

Fig. 4 shows a process for extracting calcite by an air fractionation process.

As shown, coarse pieces of limestone are stored in a first hopper 10, and the coarse pieces are further crushed by a crusher 12.

The thus-crushed pieces are temporarily stored in a second hopper 14, and are gradually discharged into a fractionating device 16 through the outlet formed in the bottom of the second hopper 14.

A sirocco fan 18 is disposed on one side of the fractionating device 16 (on the right-hand side in Fig. 4). The crushed pieces discharged from the side of the bottom of the second hopper 14 is blown off by the air sent by the fan 18 toward collecting vessels 20, 22 and 24 disposed in a lower portion of the left-hand side of the fractionating device 16.

Accordingly, particles with coarse particle sizes and high specific gravity collect in the collecting vessel 20 disposed in the vicinity of the fan 18, particles with medium particle sizes and intermediate specific gravity collecting in the collecting vessel 22, and particles with fine particle sizes and low specific gravity collecting in the collecting vessel 24 disposed away from the fan 18.

Since calcite derived from glacial limestone has light specific gravity and a fine particle diameter, it finally collects in the collecting vessel 24, and the greater part of the remaining components collect in the collecting vessels 20 and 22.

As described above, use of the illustrated apparatus enables extraction of calcite from glacial limestone without involving any changes in shape nor quality.

Table 1 shows the distribution of particle sizes of the thus-extracted calcite.

## TABLE 1

| Particle Size (Mesh) | Distribution (%) | | | |
|---|---|---|---|---|
| | A | B | C | D |
| 42 or less | 3.2 | 0.0 | 0.0 | 1.7 |
| 42 – 60 | 10.7 | 3.5 | 4.5 | 1.0 |
| 60 – 80 | 12.2 | 5.8 | 16.0 | 5.0 |
| 80 – 100 | 42.0 | 36.3 | 15.4 | 57.7 |
| 100 – 150 | 18.7 | 28.0 | 16.5 | 19.4 |
| 150 – 200 | 7.7 | 14.7 | 18.8 | 7.5 |
| 200 or more | 5.5 | 11.7 | 28.8 | 7.7 |

In Table 1, A, B, C and D represent test sections.

It will be understood from Table 1 that calcite drived from glacial limestone produced in Iran is extremely fine.

Referring to Fig. 5 showing another process for extracting calcite by precipitation, like parts are identified by like reference numerals, and the detailed descriptions are omitted for the sake of simplicity.

As shown, limestone is crushed by the crusher 12, and is stored in a vessel 50.

A stirring rod 54 connected at its one end to a motor 52 projects upwardly through the bottom of the vessel 50. The crushed pieces stored in the vessel 50 is stirred together with water for about thirty minutes.

In consequence, a suspension of the crushed pieces is produced in the vessel 50.

After the suspension has been completely stirred, the motor 52 is stopped and the suspension is placed in a stationary manner for about thirty minutes. In consequence, although precipitates are obtained in a lower portion of the vessel 50, the greater part of calcite remains in supernatant liquid because of its fine particle size and low specific gravity.

A pipe 56 is disposed in a side wall of the vessel 50, and the supernatant liquid is collected through the pipe 56.

It is to be noted that the above-described suspending and fractionating steps are preferably repeated at least three times in order to fractionate the calcite component to a satisfactory extent.

The main solid component of the supernatant liquid is granular calcite, and the desired calcite can be extracted by eliminating water by heat drying.

Table 2 shows the distribution of particle sizes of the calcite thus obtained by the aforesaid precipitation.

### TABLE 2

| Particle Size | Distribution (%) | |
| --- | --- | --- |
| (Mesh) | A | B |
| 35 or less | 8.1 | 0.0 |
| 35 - 42 | 2.0 | 0.0 |
| 42 - 60 | 6.5 | 4.5 |
| 60 - 80 | 2.5 | 16.0 |
| 80 - 100 | 10.0 | 15.4 |
| 100 - 150 | 29.9 | 16.5 |
| 150 - 200 | 26.8 | 18.8 |
| 200 or more | 14.2 | 28.8 |

It will be understood from Table 2 that the calcite extracted through precipitation is also extremely fine.

PRODUCTION AND USE OF SCRUB TYPE OF CLEANSING PREPARATION

The aforesaid scrubbing agent is preferably made into a cleansing preparation in the following manner.

In a first method, the aforesaid scrubbing agent is placed into a blender, in which it is stirred. Beef tallow is melted at temperatures of 60°C or more and is sprayed onto the scrubbing agent, thereby obtaining a scrub type of cleansing preparation in powdered form. In this case, it is preferable to add about 3% beef tallow to a 97% scrubbing agent. In place of the beef tallow, it is also suitable to use mutton tallow, vaseline, horse oil, deep-see whale oil, olive oil, jojoba oil or lauric derivatives.

4 g of the cleansing preparation thus produced is added to cold or hot water in the same amount. After they have been sufficiently mixed with each other between the palms of hands, the mixture is applied to a face or a neck and massage is performed thereon.

In a second method, while water is being sprayed on the aforesaid scrubbing agent, they are mixed with each other. After the water is uniformly mixed with the scrubbing agent to a sufficient extent, heat-melted beef tallow is sprayed thereon. Subsequently, the mixture is rounded to an appropriate size, and is dried under the sun or in a drying oven, thereby obtaining a scrub type of cleansing preparation. In this case, it is preferable to add about 3% beef tallow and about 13% water to a 84% scrubbing agent.

The cleansing preparation thus produced is applied to a face or a neck, and massage is performed by hands or fingers moistened with water.

In a third method, the aforesaid scrubbing agent is placed into a blender, in which it is stirred. Cleansing cream is added to the scrubbing agent, and they are sufficiently stirred and mixed with each other. In this case, it is preferable to add about 15% cleansing cream to a 85% scrubbing agent.

The cleansing preparation thus produced is applied to a face or a neck, and massage is performed by hands or fingers moistened with water.

Since the present scrubbing agent is difficult to dissolve in water, it can be easily removed.

It was also confirmed that, if sodium chloride, sodium sulfate and borax were mixed with the cleansing preparation in an amount equivalent to 0.05 to 5% of the latter, the water retentativity of the cleansing preparation is improved and thus the productivity and usability were also improved. In addition, the cleansing preparation became smooth to the touch.

The water content of the scrubbing agent is preferably 2 to 5%. When the water content is 2% or less, the product easily becomes rough to the touch while, when it is 5% or more, the product becomes sticky to the touch and difficult to handle. It is considered that the smoothness is attributed to the astringency of skin which may be influenced by sodium chloride.

An extending agent for the scrubbing agent is preferably selected from the group consisting of anhydrous silicic acid, kaolin, magnesium silicate and the like, and it is possible to add the extending agent in an amount equivalent to 10%.


RESULT OF TEST

The advantages of the cleansing preparation containing the scrubbing agent of the invention as the main raw material will be described below with reference to the results of panel tests.

Table 3 shows the result of comparison between the present scrubbing type of cleansing preparation and the conventional one. The panel includes men and women in their teens to sixteens.


TABLE 3

When the inventive product is compared with the conventional product, the former is:
Superior      ; 68 (persons)
Superior to some extent    ; 24
Substantially the same    ; 8
Inferior to some extent    ; 0
Inferior    ; 0

In addition, the following opinions were obtained through the comparative test.

a) The inventive product has an effect equivalent to a combination of massaging cream and a pack. After use, skin is maintained in a suitably wet state. The following morning, cosmetics spread well over skin, thereby enabling a comfortable make-up.

b) Although I forgot to remove my make-up before sleeping, I did not suffer from any skin irritations the next morning, and cosmetics spread well.

c) In spite of an allergic constitution, no allergic reaction appears, and cosmetics spread well.

d) The inventive product is particularly effective with respect to the corners of eyes, both sides of a nose, the backs of ears, a chin or other portions in which fat or wastes easily remain.

f) I frequently suffered from spots on the skin because of my rough skin. However, the inventive product enables comfortable massage and firm skin without any stretch.

g) Although a slight pain is felt due to excessive massage, cosmetics spread well over skin.

h) Cosmetics spread well irrespective of shortage of sleep.

i) It is possible to completely and quickly remove cosmetics (foundations or the like) which are normally difficult to remove merely with a soap and water.

8

k) When I cleanse my face with soap and water, I occasionally experience a rash. When I tried the inventive product, a slight pain was felt. However, the following morning, the pain already disappeared and cosmetics spread well on my skin.

l) I commonly uses special kinds of cosmetics because of my heavy pollinosis and allergic rhinitis. However, the inventive product was suitable for use on my skin and cosmetics spread well on my skin.

m) The inventive product was applied to a circular bald spot attributed to alopecia nerotica, continuously for about one month. As a result, hair started to grow there.

Incidentally, in a case where powdered calcite produced in Iran was used in the form of a simple substance, a satisfactory scrubbing effect was likewise achieved. In particular, such a product was favorably received by the female panel.

It is also reported that the inventive product is effective in removing spots.

As described above, various advantages are reported in connection with the scrub type of cleansing preparation of the present invention, but no problem is reported.

As will be readily understood from the foregoing, the scrubbing agent in accordance with the present invention contains calcite extracted from glacial limestone. Accordingly, even a user with an allergic constitution can enjoy suitable cleansing and massage effects without experiencing the skin irritations.

In addition, the process for producing the scrubbing agent in accordance with the present invention comprises the step of extracting calcite from glacial limestone by air fractionation or precipitation. Accordingly, it is possible to obtain scrubbing agents containing calcite as the main component, without affecting the particle diameter and configuration of calcite in glacial limestone.

## Claims

1. A scrubbing agent comprising granulated calcite extracted from glacial limestone.

2. A scrubbing agent according to Claim 1, wherein said granulated calcite essentially contains particles having a particle size of 10 $\mu$m or less.

3. A scrubbing agent according to Claim 1 or 2, wherein said granulated calcite comprises the following particle size composition:

      32 - 42    ; Me 0.0 - 15.0%
      42 - 60 Me   ; 0.0 - 15.0%
      60 - 80 Me   ; 0.0 - 20.0%
      80 - 100 Me   ; 10.0 - 60.0%
      100 - 150 Me   ; 15.0 - 35.0%
      150 - 200 Me   ; 5.0 - 40.0%
      200 Me or more   ; 5.0 - 30.0%

4. A scrubbing agent according to Claim 1 or 2, wherein said scrubbing agent has a pH which is adjusted in the range of from neutrality to weak acidity with the use of one of organic acid and fatty acid.

5. A scrubbing agent according to Claim 3, wherein said scrubbing agent has a pH which is adjusted in the range of from neutrality to weak acidity with the use of one of organic acid and fatty acid.

6. A process for producing a scrubbing agent containing calcite as the main component, comprising:

the crushing step of crushing glacial limestone; and

the fractionating step of subjecting said glacial limestone in said crushing step to air fractionation to extract a fractionated portion containing calcite as the main component.

7. A process for producing a scrubbing agent containing calcite as the main component, comprising:

the crushing step of crushing glacial limestone;

the suspending step of suspending in water said glacial limestone crushed in said crushing step;

the fractionating step of placing a suspension obtained in said suspending step in a stationary manner to collect supernatant liquid thus produced; and

the drying step of drying said supernatant liquid.

8. The process according to Claim 7, wherein stirring is performed in said suspending step for thirty to forty minutes.

9. The process according to Claim 7 or 8, wherein said suspension is placed in a stationary manner in said fractionating step for twenty to forty minutes.

10. The process according to Claim 7 or 8, wherein said suspending step and said fractionating step are repeated a plurality of times.

11. The process according to Claim 9, wherein said suspending step and said fractionating step are repeated a plurality of times.

9

# F I G. 1

$\times 10^4$

CaCO₃

0 257 458

0 257 458

FIG. 2A

FIG. 2B

FIG. 3 A

0003    5KV    X1,000    10μm WD19

FIG. 3 B

0004    5KV    X4,000    1μm WD19

# FIG.4

# FIG.5